# EUROPEAN PATENT SPECIFICATION

(11) **EP 4 135 652 B1**
(45) Date of publication and mention of the grant of the patent: **18.12.2024**
(21) Application number: 21717110.7
(22) Date of filing: 13.04.2021
(51) Int. Cl.: A61K 9/00, A61K 9/19, A61K 31/357, A61K 47/10, A61K 47/22, A61K 47/24, A61K 47/26, A61K 47/44

(54) **RESINIFERATOXIN COMPOSITIONS**
RESINIFERATOXINZUSAMMENSETZUNGEN
COMPOSITIONS DE RÉSINIFÉRATOXINE

(30) Priority: 15.04.2020 EP 20169668
(43) Date of publication of application: 22.02.2023
(73) Proprietor: Grünenthal GmbH, 52078 Aachen (DE)
(72) Inventor: BERNKOP-SCHNÜRCH, Andreas, 6080 Igls (AT); VALDENAIRE, Olivier, 4059 Basel (CH); GILLER, Thomas, 4451 Wintersingen (CH)
(74) Representative: Kutzenberger Wolff & Partner
(86) International application number: PCT/EP2021/059562
(87) International publication number: WO 2021/209450

(56) References cited:
- WO-A1-2017/087803
- WO-A1-98/56356
- US-A1- 2019 076 396
- GARDINER ET AL: "A bladder-cooling reflex in the anaesthetised guinea-pig: A model of the positive clinical ice-water test", JOURNAL OF PHARMACOLOGICAL AND TOXICOLOGICAL METHODS, ELSEVIER, NEW YORK, NY, US, vol. 55, no. 2, 1 March 2007 (2007-03-01), pages 184 - 192, XP022033517, ISSN: 1056-8719, DOI: 10.1016/J.VASCN.2006.06.003

## Description

### FIELD OF THE INVENTION

The present invention relates to the field of pharmaceutics, and concerns compositions of resiniferatoxin, their manufacture and their use.

### BACKGROUND OF THE INVENTION

Resiniferatoxin (RTX) is a naturally occurring compound produced by the Moroccan cactus *Euphorbia resinifera* and is a highly potent analog of capsaicin, the active ingredient of the red chili pepper. At very small dosages, resiniferatoxin is known as a transient receptor potential cation channel subfamily V member 1 (TRPV1) agonist, and has been investigated for therapeutic efficacy in a variety of diseases or ailments such as pain relief, overactive bladder, interstitial cystitis, and rhinitis.

Resiniferatoxin is known to easily bind to a variety of substances and surfaces, making handling of very extremely small quantities required for low dosages of the compound challenging. Moreover, when thawed it decomposes into fragments and therefore is thawed immediately before use and diluted in a solvent such as ethanol or dimethyl sulfoxide to prevent decomposition.

US 2019/076396 A discloses a non-alcoholic formulation of resiniferatoxin solubilized in a solubilizing agent, a monosaccharide or sugar alcohol, and a buffer solution.

It is thus an object of the present invention to provide compositions of resiniferatoxin that may be stored for longer periods of time without significant decomposition, in particular non-aqueous solid and liquid compositions of resiniferatoxin and a surfactant with optionally other components. The non-aqueous compositions may be diluted with a diluent such as an aqueous diluent for use in therapeutic applications. Kits, methods of manufacture, and methods of use of the resiniferatoxin compositions are also provided. Further objects of the invention will be clear on the basis of the following description of the invention, examples and claims.

### SUMMARY OF THE INVENTION

In a first aspect, the invention relates to non-aqueous solid lyophilized compositions of resiniferatoxin comprising (a) resiniferatoxin; and (b) a surfactant, wherein the surfactant is D-α-tocopherol polyethylene glycol succinate. The invention also relates to kits comprising (a) a first kit component comprising the non-aqueous solid lyophilized composition; and (b) a second kit component comprising a diluent. Also contemplated is the composition or kit for use in a method for the treatment of pain. In a specific embodiment, the pain is osteoarthritis-related joint pain.

### DETAILED DESCRIPTION OF THE INVENTION

### Definitions

The term "about" or the like in connection with an attribute or value includes the exact attribute or precise value, as well as any attribute or value typically considered to fall within the normal or accepted variability associated with the technical field, and methods of measuring or determining said attribute or value. The term "about" refers usually to a range of values ± 10% of a specified value. For example, the phrase "about 200" includes ± 10% of 200, or from 180 to 220.

The term "kit" as used herein means that the components comprised in said kit are provided physically separable and distinguishable from one another as different components but are provided or sold together for the purpose of being administered, or used, together.

The terms "lyophilized", "lyophilization", and "lyophilize" as used herein refer to a process of freeze-drying, where a sample is first frozen and dried, typically under a vacuum, to remove water and/or other solvents.

The term "non-aqueous" as used herein refers to a composition, either solid or liquid, that is substantially free of water (other than water of hydration), preferably less than 2 percent by weight of water as measured by standard techniques such as, for example, a thermogravimetric measurement. The non-aqueous compositions described herein may contain small amounts of water absorbed from ambient moisture.

The terms "resiniferatoxin" and "RTX" as used herein refer to a naturally occurring chemical produced by the Moroccan cactus *Euphorbia resinifera* or synthetically produced and has the following structure and a molar weight of 628.71:

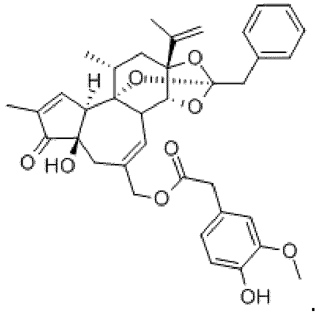

The term "D-α-tocopherol polyethylene glycol succinate" is synonymously used herein with the term "D-α-Tocopherol polyethylene glycol 1000 succinate" and the abbreviation "TPGS". D-α-tocopherol polyethylene glycol succinate is a non-ionic surfactant.

The term "solid composition" as used herein refers to solids in crystalline, powder, and/or amorphous form, or any mixtures thereof. It is in the solid state at normal temperature and pressure (NTP) as defined by the US National Institute of Standards and Technology (NIST). The solid compositions described herein may contain (in addition to any water of hydration) trace amounts of water or other solvents, preferably less than 2 wt % of the water or other solvents. The solid composition may be stored at room temperature or below, preferably about 4 °C, about 10 °C, or about 25 °C prior to reconstitution with a liquid solvent.

The term "buffer" or "buffer solution" means a combination of two or more constituents which buffer (i.e. minimize the change of) the pH value upon addition of an acid or base. Buffers or buffer solutions may further comprise auxiliary compounds, such as neutral salts like sodium chloride that contribute little to the buffer effect, but which are incorporated for physiological reasons, e.g. to adjust the osmolality of a buffer solution.

### Solid Compositions

In one aspect, the invention provides non-aqueous solid compositions of resiniferatoxin comprising resiniferatoxin and a surfactant. Optionally, the compositions comprise one or more other additives. The solid composition is a lyophilized composition, also referred to as a lyophilate or lyophilizate. The surfactant may be a non-ionic surfactant. Exemplary non-ionic surfactants include D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100. In the composition of the invention, the surfactant is D-α-tocopherol polyethylene glycol succinate.

The compositions may further include a polyethylene glycol and/or a further surfactant, where the further surfactant is optionally a poloxamer. Exemplary polyethylene glycols include PEG 1000, PEG 1450, PEG 1600, PEG 3000, PEG 3350, PEG 4000, PEG 4500, PEG 6000, PEG 8000, and PEG 20000. In some embodiments, the polyethylene glycol is PEG 6000. The poloxamer may, for example, be poloxamer 101, 182, 188, 237, 331, 338, or 407. In some embodiments, the poloxamer is poloxamer 188, 237 or 407. In another embodiment, the poloxamer is poloxamer 188.

Other optional additives include solid components of a buffer solution. The components may include, for example, NaCl, CaClz x 2 H₂O, KCl, tris(hydroxymethyl)aminomethane, and combinations thereof. In a particular embodiment, the non-aqueous solid composition comprises resiniferatoxin, D-α-tocopherol polyethylene glycol succinate, NaCl, CaClz x 2 H₂O, KCl, and tris(hydroxymethyl)aminomethane and is lyophilized. In some embodiments, the non-aqueous solid compositions disclosed herein do not comprise a monosaccharide or sugar alcohol.

The non-aqueous solid compositions of resiniferatoxin may be obtained by combining resiniferatoxin, a surfactant, and the optional additives, if any, in a solvent to form a solution or dispersion, then drying the solution or dispersion. For example, a stock solution of resiniferatoxin in an organic solvent such as absolute ethanol in a concentration of about 0.1 to about 1000 µg/mL, about 0.2 to about 200 µg/mL, or about 20 to about 50 µg/mL may be prepared. Other organic solvents that may be used include glycerin or DMSO. The stock solution (e.g., 50 µL) may be diluted with a diluent (e.g., 950 µL) containing the surfactant and optionally polyethylene glycol or a further surfactant (e.g., a poloxamer) followed by drying. In some embodiments, the diluent is an aqueous diluent. In some embodiments, the aqueous diluent comprises at least one buffering agent and optionally at least one isotonizing agent. In some embodiments, the at least one buffering agent is adapted to maintain a pH from about 7.7 to about 8.3. In other embodiments, the aqueous diluent comprises about 140 - 150 mM NaCl, about 3 - 5 mM CaCl₂ x 2 H₂O, about 3 - 5 mM KCl, about 8-12 mM tris(hydroxymethyl)aminomethane, and has a pH from about 7.7 to about 8.3. In a particular embodiment, the aqueous diluent comprises about 147 mM NaCl, about 4 mM CaClz x 2 H₂O, about 4 mM KCl, about 10 mM tris(hydroxymethyl)aminomethane, and has a pH of about 8.0.

The drying or solvent removal is carried out by lyophilization.

The non-aqueous solid compositions are typically more stable at or below room temperature than solid resiniferatoxin alone or aqueous liquid compositions of resiniferatoxin. The solids may be stored at or below room temperature before reconstitution and use. Exemplary storage temperatures include at about 0 °C, about 4 °C, or about 25 °C or within the ranges of about -5 °C to about 25 °C, about 0 °C to about 25 °C, about 0 °C to about 15 °C, or about 0 °C to about 10 °C, or about 0 °C to about 5 °C.

In some further embodiments, the non-aqueous solid compositions of the invention are characterized in that they are readily dissolvable in an aqueous carrier or diluent. In these embodiments, the solid composition may be combined with a preferably sterile aqueous diluent such that upon shaking a liquid solution is obtained which contains the resiniferatoxin in dissolved form. In some embodiments, at least 90 % of the resiniferatoxin is recovered after dissolution of the solid in an aqueous diluent. In other embodiments, at least 95 % of the resiniferatoxin is recovered after dissolution of the solid in an aqueous diluent. In some embodiments, at least 90% of the resiniferatoxin is recovered after storage at about -5 °C to about 25 °C, about 0 °C to about 25 °C, about 0 °C to about 15 °C, or about 0 °C to about 10 °C, or about 0 °C to about 5 °C for up to about 1 to about 24 months, about 6 to about 24 months, or about 12 to about 24 months. In a particular embodiment, at least 90% of the resiniferatoxin is recovered after storage at about 0 °C to about 25°C for up to about 3 to about 24 months. In another particular embodiment, at least 95% of the resiniferatoxin is recovered after storage at about 0 °C to about 25°C for up to about 3 to about 12 months.

### Kits

In another aspect, the invention provides a kit comprising (a) a first kit component comprising the non-aqueous solid composition as described herein and (b) a second kit component comprising a diluent.

In some embodiments, the diluent may be an aqueous solution or an organic solvent. Exemplary organic solvents include absolute ethanol, glycerin, and dimethyl sulfoxide (DMSO). A particular diluent is an aqueous diluent. The aqueous diluent may comprise only water or may comprise at least one buffering agent and optionally at least one isotonizing agent. The buffering agent may be adapted to maintain a pH from about 7.7 to about 8.3 upon combining the first and second kit components. In some embodiments, the aqueous diluent comprises about 140 - 150 mM NaCl, about 3 - 5 mM CaCl₂ x 2 H₂O, about 3 - 5 mM KCl, about 8 - 12 mM tris(hydroxymethyl)aminomethane, and has a pH from about 7.7 to about 8.3. In a particular embodiment, the aqueous diluent comprises about 147 mM NaCl, about 4 mM CaCl₂ x 2 H₂O, about 4 mM KCl, about 10 mM tris(hydroxymethyl)aminomethane, and has a pH of about 8.0.

In some embodiments, the first kit component is the non-aqueous solid lyophilized composition comprising resiniferatoxin, a surfactant, wherein the surfactant is D-α-tocopherol polyethylene glycol succinate, and optionally at least one buffering agent and optionally at least one isotonizing agent, and the second kit component is water. In some embodiments, the buffering agent and optional isotonizing agent comprise NaCl, CaCl₂ x 2 H₂O, KCl, and tris(hydroxymethyl)aminomethane. In certain embodiments, when the first kit component (non-aqueous solid composition) is combined with the second kit component (water), the resulting aqueous solution has a pH from about 7.7 to about 8.3.

In some embodiments, the kit may be supplied in the form of:
(a) separate compartments of one primary package (such as a sachet divided into two or more 'sub-pouches' by a laminating seam, or a glass vial filled with one kit component and the other kit component being held in the screw-top lid of said glass vial);
(b) separate primary packages packaged together within one secondary package (such as separate sets of sachets or glass vials for two or more kit components, the two or more sachet-sets or glass vial-sets being sold in one and the same folded box);
(c) separate primary packages packaged in two or more separate secondary packages which are in turn held together by paper or plastic wrappers, ribbons, sleeves or the like (such as separate sets of sachets or glass vials for two or more kit components, the two or more sachet-sets or glass vial-sets being sold in two or more cardboard boxes, the latter being wrapped with a shrink foil wrapper); or
(d) combinations thereof (such as a first kit-component being provided in multiple-dose cardboard drum, optionally with a dosing spoon, the cardboard drum being sold in a folded box together with a multitude of glass vials, each glass vial containing a second kit-component).

Optionally, the kits of the invention may further comprise written instructions on how to best, or preferably, combine and use the two or more kit components.

### Aqueous compositions for injection and use

Also disclosed, but not forming part of the presently claimed invention, are aqueous compositions of resiniferatoxin comprising (a) resiniferatoxin (b) a surfactant (c) and at least one buffering agent, wherein the composition has a pH from about 7.7 to about 8.3. The aqueous compositions may be prepared by combining the first and second kit components as described above. In some embodiments, the aqueous compositions comprise (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01 vol % to about 10 vol % of a surfactant, preferably from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant; and (c) at least one buffering agent; wherein the composition has a pH from about 7.7 to about 8.3. In other embodiments, the aqueous compositions comprise (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01 vol % to about 10 vol % of a surfactant, preferably from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant; (c) about 140 - 150 mM NaCl; (d) about 3 - 5 mM CaCl₂ x 2 H₂O; (e) about 3 - 5 mM KCl; (f) and about 8 - 12 mM tris(hydroxymethyl)aminomethane; wherein the composition has a pH from about 7.7 to about 8.3. In particular embodiments, the aqueous compositions comprise (a) from about 0.01 µM to about 1 µM resiniferatoxin, preferably from about 0.1 µM to about 1 µM resiniferatoxin, more preferably from about 0.1 µM to about 0.2 µM resiniferatoxin; (b) from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant0.35 vol % of a surfactant; (c) about 147 mM NaCl; (d) about 4 mM CaCl₂ x 2 H₂O; (e) about 4 mM KCl; (f) and about 10 mM tris(hydroxymethyl)aminomethane; wherein the composition has a pH of about 8.0. In particular embodiments, the aqueous compositions do not comprise a monosaccharide or sugar alcohol.

### Methods of Use

The non-aqueous solid compositions, and/or the aqueous composition of resiniferatoxin disclosed herein may be used for the manufacture of a medicament for the treatment of pain. Thus the present invention provides the non-aqueous solid lyophilized compositions of resiniferatoxin disclosed herein for use as a medicament. In some embodiments, the compositions disclosed herein are used for the treatment of pain. Thus the present invention provides the non-aqueous solid lyophilized compositions of resiniferatoxin disclosed herein for use in a method for the treatment of pain. In particular embodiments, the pain is osteoarthritis-related joint pain.

The following examples serve to illustrate the invention, however should not be understood as restricting the scope of the invention.

### EXAMPLES

### Materials

The following materials were used to prepare exemplary non-aqueous solid and liquid compositions described herein: acetonitrile (Lot: STBH6791, Sigma-Aldrich, Austria); ammonium formate (Lot: BCBD3147V, Sigma-Aldrich, Austria); calcium chloride dihydrate (Lot: S28238-156, Sigma-Aldrich, Austria); Cremophor EL (Lot: 86741436W0, BASF; Germany); Cremophor RH60 (Lot: 91191656P0, BASF; Germany); formic acid (Lot: 396236476, Carl Roth, Karlsruhe, Germany); hydrochloric acid (Lot: H2116K1, Carl Roth, Germany); mannitol (Lot: 1560453, Thermo Scientific, Austria); ortho - phosphoric acid 85% for HPLC (Lot: Z0440928, Merck, Germany); PEG6000 (polyethylene glycol 6000; Lot: 2005TS1586, Croda; Germany); potassium chloride (Lot: 351173748, Carl Roth, Germany); RTX solution 10 mg·mL⁻¹ provided by Mestex (Lot: LIMS200949145, Carbogen Amcis, Switzerland); sodium chloride (Lot: 177255242, Carl Roth, Germany); sodium phosphate monobasic monohydrate (Lot: BCBG8983V, Sigma-Aldrich, Austria); TPGS (D-α-Tocopherol polyethylene glycol 1000 succinate ; Lot: BCBX8795, Sigma-Aldrich, Austria); tris (tris(hydroxymethyl)aminomethane, Trizema Ph. Eur, (Lot: S28238-156, Sigma-Aldrich, Vienna, Austria); and Tween 80 (polysorbate 80, Lot: K47176961 602, Merck Millipore, Vienna, Austria).

### Example 1

### Preparation of non-aqueous solid RTX compositions

A stock solution of 1 mg/ml RTX in EtOH abs. was prepared. From this stock solution, working solutions were prepared containing the desired RTX concentration in EtOH. Test vials were prepared by combining 50 µl of RTX working solution with 950 µl of Buffer containing the corresponding surfactants and/or poloxamers. All prepared vials were homogenized, covered with Parafilm, frozen and subsequently lyophilized to produce the non-aqueous solid RTX compositions.

### Exemplary Compositions

Exemplary non-aqueous solid compositions that have been prepared are summarized in Table 1 below. Only the compositions comprising TPGS are according to the invention.

**Table 1**

| | Lyophilized RTX/ethanol/Tween/ PEG6000/Mestex buffer | Lyophilized RTX/ethanol/TPGS/ Mestex buffer | Lyophilized RTX/ethanol/TPGS/ PEG6000/Mestex buffer |
|---|---|---|---|
| Composition | 3.2 µg/vial RTX 70 mg/vial Tween PEG6000 1x Mestex buffer | 3.2 µg/vial RTX 70 mg/vial TPGS 1x Mestex buffer | 3.2 µg/vial RTX 70 mg/vial TPGS PEG6000 1x Mestex buffer |
| Condition | Lyophilized | Lyophilized | Lyophilized |
| Preparation of final formulation | Reconstitution with water for injection | Reconstitution with water for injection | Reconstitution with water for injection |
| Storage conditions | optional at 4°C and 25°C/60% RH glass vials | 4°C and 25°C/60% RH glass vials | 4°C and 25°C/60% RH glass vials |
| Sampling | optional 1 m, 2 m, 3 m, 6 m, 9 m and 12 m | 1 m, 2 m and 3 m optional 6 m, 9 m and 12 m | 1 m, 2 m and 3 m optional 6 m, 9 m and 12 m |

Table 1 shows non-aqueous solid compositions prepared. "Mestex buffer" (MB) contains 147 mM NaCl, 4 mM CaClz x 2 H₂O, 4 mM KCl, 10 mM tris(hydroxymethyl)aminomethane, and has a pH of about 8.0.

### Stability Tests

For certain exemplary non-aqueous compositions described herein, the % values of the amount of resiniferatoxin detected by HPLC after lyophilization and reconstitution are provided in Table 2, below. After the lyophilization process, all vials were sealed and stored at the corresponding conditions as shown in Table 1. At each time point (month (m)), the required number of vials were reconstituted with 1 mL of water for injection by gently shaking the vials until complete dissolution of the lyophilized product was achieved. After 5 min, samples were taken, diluted 1:1 with ethanol and analyzed by HPLC. Moreover, a calibration curve was established at each time point using the RTX dilutions made from the stock solution in order to ensure comparability of the obtained data. Only the compositions comprising TPGS are according to the invention.

**Table 2**

| | TPGS/MB | | TPGS/PEG/MB | | Tween/PEG/MB | | Creph. 40/MB | | Tween/MB | | | Creph. E/Mannitol | |
|---|---|---|---|---|---|---|---|---|---|---|---|---|---|
| | 4°C | 25°C | 4°C | 25°C | 4°C | 25°C | 4°C | 25°C | 4°C | 40°C^{C} | 40°C | 4°C | 25°C |
| T0 | | | | | | | | | | 101.0 | | | 102.0 |
| 1 m | 94.2 | 92.8 | 96.2 | 92.3 | 100.3 | 94.2 | | | 97.2 | 81.0 | 56.8 | | |
| 2 m | 96.5 | 94.2 | 93.5 | 88.9 | 99.5 | 90.9 | | | 89.5 | 57.0 | 52.6 | | |
| 3 m | 97.9 | 95.9 | 90.3 | 91.3 | | 87.7 | | 93.0 | 86.2 | 55.0 | 60.5 | | |
| 6 m | 97.9 | 91.4 | | | 88.1 | 88.0 | | | | | | | |
| 9 m | 93.3 | 88.0 | | | 85.1 | 88.0 | | | | | | | |
| 12 m | 87.6 | 83.7 | | | 85.3 | n.d. | | | | | | | |

Table 2 shows stability data of non-aqueous solids over 12 months. All reconstituted solutions were clear and colorless. The pH was 8.0 ± 0.2.

### Example 2 (not according to the invention)

### Preparation of non-aqueous liquid RTX compositions

A aseptic solution with the desired concentrations of RTX and the corresponding surfactant and/or poloxamers in EtOH abs. was prepared. 0.5ml of this solution is distributed into 10R vials and sealed with stoppers and crimped. Such preparations are stored at 5°C.

Exemplary non-aqueous liquid compositions that have been prepared are summarized in Table 3 below.

In addition to the non-aqueous solid compositions RTX was also formulated in EtOH (abs) together with different surfactants as liquid pre-concentrates. At each time point the content of the vial was diluted 1 to 20 with Mestex buffer without the surfactants. After 5 min, samples were taken, diluted 1:1 with ethanol and analyzed by HPLC. Moreover, a calibration curve was established at each time point using the RTX dilutions made from the stock solution in order to ensure comparability of the obtained data.

In table 3 below the results of RTX stability in such compositions are shown as percentage of the T0 value which was measured after an initial filtration and centrifugation.

**Table 3**

| | Tween¹/EtOH | | Tween²/EtOH | | | Creph. EL/EtOH | | | Creph. RH60/EtOH | | | Creph. E/ Mannitol | | Lipoid S100 | | Lipoid S100/ Mannitol | |
|---|---|---|---|---|---|---|---|---|---|---|---|---|---|---|---|---|---|
| | 4°C | 25°C | 4°C | 25°C | 40°C | 4°C | 25°C | 40°C | 4°C | 25°C | 40°C | 4°C | 25°C | 4°C | 25°C | 4°C | 25°C |
| ⁻0 | 95.0 | 95.0 | 100.0 | 100.0 | 100.0 | 100.0 | 100.0 | 100.0 | 100.0 | 100.0 | 100.0 | | 102.0 | | 66.0 | | 70.0 |
| 1m | 97.3 | 92.8 | | | | | | | | | | | | | | | |
| 2m | 93.4 | 77.1 | | | | | | | | | | | | | | | |
| 8 m | 94.3 | 77.7 | 100.0 | 100.0 | 90.0 | 100.0 | 100.0 | 90.0 | 100.0 | 100.0 | 88.0 | | | | | | |
| 6 m | | | 100.0 | 100.0 | | 100.0 | 100.0 | | 100.0 | 100.0 | | | | | | | |
| 9 m | | | | | | | | | | | | | | | | | |
| 12 m | | | 100.0 | 100.0 | | 95.0 | 100.0 | | 100.0 | 100.0 | | | | | | | |

| | | | | | | | | | | | | | | | | | |
|---|---|---|---|---|---|---|---|---|---|---|---|---|---|---|---|---|---|
| 1=TWEEN 14% 2=Tween 0.08% | | | | | | | | | | | | | | | | | |

Table 3 shows stability data of non-aqueous liquid compositions. All reconstituted solutions were clear and colorless.

All the non-aqueous liquid compositions still contain 5% solvent and together with the fact that for such formulations an extra vial with the corresponding buffer has to be prepared and delivered, the use of non-aqueous liquid formulations is less preferred compared to the non-aqueous solid compositions described in here.

### Example 3

### Rehydration of non-aqueous solid RTX compositions and short term stability

An appropriate amount of water for injection (WFI) was added to a vial containing the lyophilized RTX composition as prepared in Example 1 comprising TPGS/Mestex Buffer ("TPGS/MB" as designated in Table 2 above at three different concentrations 0.1 %, 0.35 % and 0.7 %) to prepare the ready to use medication for intra-articular injection into joints. As can be seen from Table4 below, the rehydrated lyophilized RTX compositions are stable over the whole measured period of four hours independent of the TPGS concentrations used.

**Table 4**

| | TPGS concentration in final mixture | | | | | |
|---|---|---|---|---|---|---|
| | 0.10% | | 0.35% | | 0.70% | |
| | 4°C | 25°C | 4°C | 25°C | 4°C | 25°C |
| T0 | 100±2.1 | | 100±5.8 | | 100.0±2.6 | |
| 1h | 101.9 ±2.2 | 101.5±3.7 | 107.1±2.7 | 101.4±1.5 | 98.3±2.8 | 96.8±7.7 |
| 2h | 102.7 ±5.3 | 100.2±3.7 | 105.2±1.9 | 101.0±2.5 | 100.4±4.9 | 99.3±8.5 |
| 4h | 101.9±3.3 | 100.1±1.9 | 103.5±1.2 | 101.9±2.2 | 98.6±6.0 | 96.9±7.2 |

Table 4 shows results of stability studies of the formulation RTX/EtOH/0.10, 0.35 or 0.70 % TPGS/Mestex buffer. Samples were diluted 1:1 with EtOH before HPLC analysis.

Indicated values are means (n ≥ 3) ± SD.

## Claims

1. A non-aqueous solid lyophilized composition of resiniferatoxin, the composition comprising:
(a) resiniferatoxin; and
(b) a surfactant, wherein the surfactant is D-α-tocopherol polyethylene glycol succinate.

2. The composition of claim 1, wherein at least 90 % of the resiniferatoxin is recovered after dissolution of the solid in an aqueous diluent.

3. The composition of any one of claim 1 or 2, wherein at least 90% of the resiniferatoxin is recovered after storage at about 0 °C to about 25 °C for up to about 3 to about 24 months.

4. The composition of any one of claims 1 to 3, wherein the composition comprises resiniferatoxin, D-α-tocopherol polyethylene glycol succinate, NaCl, CaCh x 2 H₂O, KCl, and tris(hydroxymethyl)aminomethane.

5. The composition of any one of claims 1 to 4, wherein the composition does not comprise a monosaccharide or sugar alcohol.

6. A kit comprising:
(a) a first kit component comprising the composition of any one of claims 1 to 5; and
(b) a second kit component comprising a diluent.

7. The kit of claim 6, wherein the diluent is an aqueous solution.

8. The kit of any one of claims 6 to 7, wherein when the first kit component is combined with the second kit component, the resulting aqueous solution has a pH from about 7.7 to about 8.3.

9. The kit of any one of claims 6 to 8, wherein the diluent is aqueous and comprises at least one buffering agent and optionally at least one isotonizing agent.

10. The kit of claim 9, wherein the buffering agent is adapted to maintain a pH from about 7.7 to about 8.3 upon combining the first and second kit components.

11. The kit of any one of claims 6 to 10, wherein the diluent is aqueous and comprises about 140 - 150 mM NaCl, about 3 - 5 mM CaCl₂ x 2 H₂O, about 3 - 5 mM KCl, about 8 - 12 mM tris(hydroxymethyl)aminomethane, and has a pH from about 7.7 to about 8.3.

12. The kit of any one of claims 6 to 11, wherein the kit is supplied in the form of:
(a) separate compartments of one primary package;
(b) separate primary packages packaged together within one secondary package;
(c) separate primary packages packaged in two or more separate secondary packages which are in turn held together by paper or plastic wrappers, ribbons, sleeves or the like; or
(d) combinations thereof.

13. The composition of any one of claims 1 to 5 or the kit of any of claims 6 to 12 for use in a method for the treatment of pain.

## Patentansprüche

1. Nichtwässrige, feste, lyophilisierte Zusammensetzung von Resiniferatoxin, wobei die Zusammensetzung Folgendes umfasst:
(a) Resiniferatoxin; und
(b) ein Tensid, wobei das Tensid D-α-Tocopherolpolyethylenglykolsuccinat ist.

2. Zusammensetzung nach Anspruch 1, wobei mindestens 90 % des Resiniferatoxins nach dem Auflösen des Feststoffs in einem wässrigen Verdünnungsmittel wiedergewonnen werden.

3. Zusammensetzung nach einem der Ansprüche 1 oder 2, wobei mindestens 90 % des Resiniferatoxins nach Lagerung bei etwa 0 °C bis etwa 25 °C für bis zu etwa 3 bis etwa 24 Monate wiedergewonnen werden.

4. Zusammensetzung nach einem der Ansprüche 1 bis 3, wobei die Zusammensetzung Resiniferatoxin, D-α-Tocopherolpolyethylenglykolsuccinat, NaCl, CaCl₂ x 2 H₂O, KCl und Tris(Hydroxymethyl)aminomethan umfasst.

5. Zusammensetzung nach einem der Ansprüche 1 bis 4, wobei die Zusammensetzung kein Monosaccharid oder keinen Zuckeralkohol umfasst.

6. Kit, umfassend:
(a) eine erste Kit-Komponente, die die Zusammensetzung nach einem der Ansprüche 1 bis 5 umfasst; und
(b) eine zweite Kit-Komponente, die ein Verdünnungsmittel umfasst.

7. Kit nach Anspruch 6, wobei das Verdünnungsmittel eine wässrige Lösung ist.

8. Kit nach einem der Ansprüche 6 bis 7, wobei, wenn die erste Kit-Komponente mit der zweiten Kit-Komponente kombiniert wird, die resultierende wässrige Lösung einen pH-Wert von etwa 7,7 bis etwa 8,3 hat.

9. Kit nach einem der Ansprüche 6 bis 8, wobei das Verdünnungsmittel wässrig ist und mindestens ein Puffermittel und gegebenenfalls mindestens ein Isotonisierungsmittel umfasst.

10. Kit nach Anspruch 9, wobei das Puffermittel so angepasst ist, bei dem Kombinieren der ersten und zweiten Kit-Komponente einen pH-Wert von etwa 7,7 bis etwa 8,3 aufrechtzuerhalten.

11. Kit nach einem der Ansprüche 6 bis 10, wobei das Verdünnungsmittel wässrig ist und etwa 140 - 150 mM NaCl, etwa 3 - 5 mM CaCl₂ x 2 H₂O, etwa 3 - 5 mM KCl, etwa 8 - 12 mM Tris(Hydroxymethyl)aminomethan umfasst und einen pH-Wert von etwa 7,7 bis etwa 8,3 hat.

12. Kit nach einem der Ansprüche 6 bis 11, wobei der Kit geliefert wird in Form von:
(a) getrennten Fächern einer Primärverpackung;
(b) getrennten Primärpackungen, die zusammen in einer Sekundärpackung verpackt sind;
(c) getrennten Primärpackungen, die in zwei oder mehr getrennten Sekundärpackungen verpackt sind, die jeweils durch Papier- oder Kunststoffumschläge, Bänder, Hüllen oder dergleichen zusammengehalten werden; oder
(d) Kombinationen davon.

13. Zusammensetzung nach einem der Ansprüche 1 bis 5 oder Kit nach einem der Ansprüche 6 bis 12 zur Verwendung in einem Verfahren zur Behandlung von Schmerzen.

## Revendications

1. Composition lyophilisée solide non aqueuse de résinifératoxine, la composition comprenant :
(a) de la résinifératoxine ; et
(b) un tensioactif, dans laquelle le tensioactif est le succinate de D-α-tocophérol de polyéthylène glycol.

2. Composition selon la revendication 1, dans laquelle au moins 90 % de la résinifératoxine est récupérée après dissolution du solide dans un diluant aqueux.

3. Composition selon l'une quelconque des revendications 1 ou 2, dans laquelle au moins 90 % de la résinifératoxine est récupérée après stockage à une température d'environ 0 °C à environ 25 °C pendant jusqu'à environ 3 à environ 24 mois.

4. Composition selon l'une quelconque des revendications 1 à 3, dans laquelle la composition comprend de la résinifératoxine, du succinate de D-α-tocophérol de polyéthylène glycol, du NaCl, du CaCl₂ x 2 H₂O, du KCl et du tris(hydroxyméthyl)aminométhane.

5. Composition selon l'une quelconque des revendications 1 à 4, dans laquelle la composition ne comprend pas ni monosaccharide ni d'alcool de sucre.

6. Kit comprenant :
(a) un premier composant de kit comprenant la composition selon l'une quelconque des revendications 1 à 5 ; et
(b) un second composant de kit comprenant un diluant.

7. Kit selon la revendication 6, dans lequel le diluant est une solution aqueuse.

8. Kit selon l'une quelconque des revendications 6 et 7, dans lequel lorsque le premier composant de kit est combiné avec le second composant de kit, la solution aqueuse résultante a un pH d'environ 7,7 à environ 8,3.

9. Kit selon l'une quelconque des revendications 6 à 8, dans lequel le diluant est aqueux et comprend au moins un agent tampon et éventuellement au moins un agent isotonisant.

10. Kit selon la revendication 9, dans lequel l'agent tampon est adapté pour maintenir un pH d'environ 7,7 à environ 8,3 lors de la combinaison des premier et second composants de kit.

11. Kit selon l'une quelconque des revendications 6 à 10, dans lequel le diluant est aqueux et comprend environ 140 à 150 mM de NaCl, environ 3 à 5 mM de CaCl₂ x 2 H₂O, environ 3 à 5 mM de KCl, environ 8 à 12 mM de tris(hydroxyméthyl)aminométhane, et a un pH d'environ 7,7 à environ 8,3.

12. Kit selon l'une quelconque des revendications 6 à 11, dans lequel le kit est fourni sous la forme :
(a) de compartiments séparés d'un même emballage primaire ;
(b) d'emballages primaires distincts emballés ensemble dans un emballage secondaire ;
(c) d'emballages primaires séparés emballés dans deux emballages secondaires distincts ou plus qui sont à leur tour maintenus ensemble par des emballages en papier ou en plastique, des rubans, des manchons ou analogues ; ou
(d) de combinaisons de ceux-ci.

13. Composition selon l'une quelconque des revendications 1 à 5 ou kit selon l'une quelconque des revendications 6 à 12 pour une utilisation dans un procédé de traitement de la douleur.
